# EUROPEAN PATENT APPLICATION

(11) **EP 4 134 089 A1**
(43) Date of publication of application: **15.02.2023**
(21) Application number: 21765170.2
(22) Date of filing: 27.01.2021
(51) Int. Cl.: A61K 35/744, A61P 35/00, A23L 33/135, A23K 10/16

(54) **PHARMACEUTICAL COMPOSITION COMPRISING GENUS LEUCONOSTOC STRAIN AS ACTIVE INGREDIENT FOR PREVENTION OR TREATMENT OF CANCER**

(30) Priority: 05.03.2020 KR 20200027655; 27.05.2020 KR 20200063684
(71) Applicant: Liscure Biosciences Co., Ltd., Seoul 06035 (KR)
(72) Inventor: CHIN, Hwa Sup, Yongin-si Gyeonggi-do 16824 (KR)
(74) Representative: Cabinet Becker et Associés
(86) International application number: PCT/KR2021/001060
(87) International publication number: WO 2021/177600

(57) **Abstract**

The present disclosure relates to a composition for prevention or treatment of cancer, the composition containing, as an active ingredient, a strain belonging to the genus Leuconostoc, isolated from kimchi, or a culture thereof. The genus Leuconostoc strain or the culture thereof according to the present disclosure exhibits excellent anticancer activity, and thus can be advantageously used as a composition for use in treatment, prevention, alleviation, or the like, of human or animal cancer.

## Description

### [Technical Field]

The present disclosure relates to a preventive or therapeutic composition for cancer, comprising a isolated from kimchi, specifically, a *Leuconostoc mesenteroides* LB-LM1 strain or a culture thereof as an active ingredient.

### [Background Art]

Cancer shows a high mortality rate worldwide and is the most common cause of death after cardiovascular disease in Western societies. In particular, due to the westernization of dietary habits, the intake of high-fat diets has become common, and because of a rapid increase in environmental pollutants and an increase in alcohol consumption, and the like, colon cancer, breast cancer, prostate cancer and the like continue to increase, and lung cancer is increasing due to an increase of smoking population and air pollution in addition to aging of the population. In this situation, there is an urgent need for the development of anticancer substances which can contribute to the enhancement of human health, improvement of healthy quality of life and improvement of human health by enabling early prevention and treatment of cancer.

Meanwhile, Lactobacillus is widely distributed in the oral cavity, intestine, vagina, feces of humans and animals, and fermented foods such as kimchi and are closely related to the health of humans and animals. Lactobacillus has various health-promoting effects such as bowel action, suppression of harmful bacteria, immune regulation, lowering blood cholesterol, and anticancer.

Previously, Korea Patent Publication No. 10-2010-0013184 discloses the anticancer activity of *Leuconostoc mesenteroides* strain, but only describes specific example embodiments for the preventive or therapeutic effect on colorectal cancer, and specific experimental example embodiments or implementations for other carcinomas are not given.

### [Disclosure]

### [Technical Problem]

An object of the present disclosure is to provide a pharmaceutical composition for preventing or treating cancer comprising *Leuconostoc* sp. as an active ingredient.

Another object of the present disclosure is to provide a food composition or food additive composition for preventing or improving cancer comprising *Leuconostoc* sp. as an active ingredient.

Another object of the present disclosure is to provide a feed composition or feed additive composition for preventing or improving cancer comprising *Leuconostoc* sp. as an active ingredient.

### [Technical Solution]

Accordingly, the present inventors have tried to find a lactic acid bacteria strain exhibiting cancer prevention and treatment effects from kimchi, and as a result, a novel *Leuconostoc* genus strain having anticancer activity in various carcinomas was isolated and identified to complete the present disclosure.

In order to achieve the above object, there is provided a pharmaceutical composition for preventing or treating cancer comprising *Leuconostoc* strain or a culture thereof as an active ingredient.

In addition, the present disclosure provides a food composition or food additive composition for preventing or alleviating cancer comprising *Leuconostoc* strain or a culture thereof as an active ingredient.

In addition, the present disclosure provides a feed composition or feed additive composition for preventing or alleviating cancer of livestock comprising *Leuconostoc* strain or a culture thereof as an active ingredient.

### [Advantageous Effects]

*Leuconostoc* strain or a culture thereof according to the present disclosure exhibits excellent anticancer activity against various carcinomas so that it can be usefully used as a composition for treating, preventing or alleviating human or animal cancer.

### [Description of Drawings]

FIG. 1 is a photograph showing a tumor by observing changes in the size of the tumor over time after intravenous injection of a composition according to the present disclosure to a colorectal cancer CT26 cell-transplanted mouse once.
FIG. 2 is a view showing a graph measuring the tumor growth rate over time after intravenous injection of a composition according to the present disclosure to a colorectal cancer CT26 cell-transplanted mouse once.
FIG. 3 is a view showing a graph measuring the cell growth rate (cell viability assay) of colon cancer CT26, SW620 and HCT116 cell lines treated with the composition according to the present disclosure.
FIG. 4 is a view showing a graph measuring the cell growth rate (cell viability assay) of pancreatic cancer AsPC-1 cell line treated with the composition according to the present disclosure.
FIG. 5 is a view showing a graph measuring the cell growth rate (cell viability assay) of bladder cancer T24 cell line treated with the composition according to the present disclosure.
FIG. 6 is a view showing a graph measuring the cell growth rate (cell viability assay) of melanoma B16-F10 cell line treated with the composition according to the present disclosure.
FIG. 7 is a view showing a graph measuring the cell growth rate (cell viability assay) of the normal CCD986-sk cell line treated with the composition according to the present disclosure.
FIG. 8 is a view showing a photograph taken after the colorectal cancer MC38 cell line was treated with the composition according to the present disclosure and the cell metastasis (migration assay).
FIG. 9 is a view illustrating a graph measuring the inhibitory ability of cell metastasis (migration assay) of colon cancer MC38 cell line treated with the composition according to the present disclosure.

### [Best Mode]

Hereinafter, the present disclosure is described in more detail through example embodiments. It is apparent to those of ordinary skill in the art that these example embodiments are only for illustrating the present disclosure in more detail, and the scope of the present disclosure is not limited by these example embodiments according to the gist of the present disclosure.

The composition comprising the *Leuconostoc* sp. strain of the present disclosure or a culture thereof as an active ingredient has a preventive or therapeutic effect on cancer and may be used as a pharmaceutical composition.

*Leuconostoc* sp. strain of the present disclosure is a lactic acid bacteria strain. The *Leuconostoc* strain is a probiotic and has a general intestinal effect and immune-enhancing effect of lactic acid bacteria. It is a well-known fact that lactic acid bacteria of the genus Leukonostok have an effect of enhancing intestinal effect and immunity.

The *Leuconostoc* strain may be one of *Leuconostoc mesenteroides, Leuconostoc carnosum, Leuconostoc citreum, Leuconostoc holzapfelli* and *Leuconostoc lactis.*

The *Leuconostoc mesenteroides* strain isolated from kimchi through the example embodiments of the present disclosure was found to have the nucleic acid sequence represented by SEQ ID NO: 1 as a result of 16S rRNA sequence analysis for identification and classification of microorganisms.

Therefore, the microorganism of the present disclosure having the 16S rRNA nucleotide sequence represented by SEQ ID NO: 1 was named *Leuconostoc mesenteroides* LB-LM1 and was deposited with the Korean Culture Center of Microorganisms on April 24, 2020 (Accession No. KCCM12701P).

The *Leuconostoc* strain may be used by inoculating 0.1 to 10% in MRS liquid medium and culturing at 25 to 37°C for 4 to 48 hours.

The culture method is preferably a stationary culture method, but is not limited thereto.

Herein, 'probiotics' are understood to mean living microorganisms that have a beneficial effect on health of the host by improving the host's intestinal microbial environment in the gastrointestinal tract of animals including humans. Probiotics are living microorganisms with probiotic activity and are in the form of single or complex strains, and when fed to humans or animals in the form of dried cells or fermented products to humans or animals, they can have a beneficial effect on the intestinal flora of the host.

The cancer may be any one selected from the group consisting of Bladder cancer, breast cancer, melanoma, thyroid cancer, parathyroid cancer, throat cancer, laryngeal cancer, esophageal cancer, pancreatic cancer, stomach cancer, tongue cancer, skin cancer, brain tumor, uterine cancer, head and gallbladder cancer, oral cancer, kidney cancer, liver cancer, lung cancer, colon cancer, rectal cancer and colorectal cancer, but not limited thereto.

The *Leuconostoc* sp. strain comprised in the composition according to the present invention may be present as a live cell or dead cell, and in addition, it may be present in a dried or freeze-dried form. Forms and formulation methods of lactic acid bacteria suitable for inclusion in various compositions are well known to those skilled in the art.

The composition may be administered orally or parenterally. In case of parenteral administration, it may be administered by intravenous injection, subcutaneous injection, intramuscular injection, intraperitoneal injection, endothelial administration, local administration, intranasal administration and rectal administration, and the like, and preferably, it may be administered by intravenous injection, but not limited thereto.

The appropriate dose of the present invention may be prescribed in various ways depending on factors such as formulation method, patient's age, body weight, gender, pathological condition, food, administration time, administration route, excretion rate and response sensitivity.

When the composition of the present invention is utilized as a pharmaceutical composition, the pharmaceutical composition of the present invention may be prepared by using a pharmaceutically appropriate and physiologically acceptable adjuvant in addition to the active ingredient, and as the adjuvant, an excipient, disintegrating agent, sweetener, binding agent, coating material, expansion agent, lubricant, glidant or flavoring agent, or the like may be used.

The pharmaceutical composition may be preferably formulated as a pharmaceutical composition by additionally comprising one or more kinds of pharmaceutically acceptable carriers in addition to the described active ingredient for administration.

For example, for formulation in a form of a tablet or capsule, the active ingredient may be bound to an oral and non-toxic, pharmaceutically acceptable inactive carrier, such as ethanol, glycerol, water and the like. In addition, when desired or needed, a suitable binding agent, a lubricant, a disintegrating agent and a coloring agent may also be comprised in the mixture. The suitable binding agent is not limited thereto, but includes starch, gelatin, natural sugars such as glucose or beta-lactose, natural and synthetic gum such as corn sweetener, acacia, tragacanth or sodium oleate, sodium stearate, magnesium stearate, sodium benzoate, sodium acetate, sodium chloride, and the like. The disintegrating agent is not limited thereto, but includes starch, methyl cellulose, agar, bentonite, xanthan gum, and the like. As the pharmaceutically acceptable carrier in the composition to be formulated as a liquid solution, saline solution, sterile water, Ringer's solution, buffered saline solution, albumin injection solution, dextrose solution, maltodextrin solution, glycerol, ethanol and a mixture of one or more components among them may be used, and if necessary, other common additive such as an anti-oxidant, buffer solution, a bacteriostatic agent, and the like may be added. In addition, it may be formulated as a formulation for injection such as aqueous solution, suspension, emulsion, etc., pill, capsule, granule or tablet, by additionally adding a diluent, a dispersing agent, a surfactant, a binding agent and a lubricant.

Furthermore, as an appropriate method in the field, using the method disclosed in Remington's Pharmaceutical Science, Mack Publishing Company, Easton PA, it may be preferably formulated depending on each disease or component.

The composition comprising *Leuconostoc* sp. strain or its culture as an active ingredient of the present invention may be used as a food composition or food additive composition for prevention or improvement of cancer.

The food composition may be a form of health functional food.

The "health functional food" means a food produced and processed using raw materials or ingredient having useful functions to human bodies in accordance with the Health Functional Food Act (Article 3, No. 1), and the "functionality" means to obtain useful effects for health uses such as regulating nutrients or physiological effects, and the like, on the structure and function of human bodies (same Article, No. 2).

The food composition may comprise a food additive additionally, and unless otherwise specified, the suitability as a "food additive" shall be determined according to the standards and criteria for the corresponding item in accordance with the General Rules and General Test Methods of the Food Additive Code approved by the Ministry of Food and Drug Safety.

The item listed in the "Food Additive Code" may include for example, chemical synthetic products such as ketones, glycine, potassium citrate, nicotinic acid, cinnamic acid, etc., natural additives such as Persimmon color, licorice extract, crystalline cellulose, guar gum, etc., and mixed formulations such as L-glutamine sodium formulations, alkali agents for noodles, preservative formulations, tar color formulations, etc.

The food comprising the active ingredient of the present invention may include confectionery such as bread, rice cakes, dried cakes, candies, chocolates, chewing gum and jam, ice cream produces such as ice cream, ice and ice cream powder, dairy products such as milk, low-fat milk, lactose degradation milk, processed milk, goat milk, fermented milk, butter milk, concentrated milk, milk cream, butter milk, natural cheese, processed cheese, powdered milk and whey, meat products such as processed meat products, processed egg products and hamburgers, fish meat products such as fish cakes, ham, sausage, bacon, etc., noodles such as ramen, dried noodles, fresh noodles, instant fried noodles, gelatinized dried noodles, improved cooked noodles, frozen noodles and pastas, beverages such as fruit juice beverages, vegetable beverages, soybean milk, lactobacillus beverages such as yogurt, etc., and mixed beverages, sauces such as soy sauce, soybean paste, red pepper paste, black soybean paste, mixed paste and vinegar, seasoning food such as tomato ketchup, curry and dressing, margarine, shortening and pizza, but not limited thereto.

In addition thereto, the composition of the present invention may comprise various nutrients, vitamins, electrolytes, flavoring agents, coloring agents, pectic acid and its salts, alginic acid and its salts, organic acids, protective colloid thickeners, pH adjusting agents, stabilizers, preservatives, glycerin, alcohols, carbonating agents used for carbonated beverages, and the like. Moreover, the composition of the present invention may comprise flesh for production of natural fruit juices, fruit juice beverages and vegetable beverages. These ingredients may be used independently or in combination.

The beverage composition comprising the active ingredient of the present invention has no special limitations for other components, and as common beverages, may contain an additional component such as various flavoring agents or natural carbohydrates, or the like. The example of the natural carbohydrates described above common sugars such as monosaccharides (for example, glucose, fructose, etc.); disaccharides (for example, maltose, sucrose, etc.); and polysaccharides (for example, dextrin, cyclodextrin, etc.), and sugar alcohols such as xylitol, sorbitol, erythritol, and the like. As a flavoring agent other than those described above, natural flavoring agents (thaumatin, stevia extracts (for example, rebaudioside A, glycyrrhizin, etc.)) and synthetic flavoring agents (saccharin, aspartame, etc.) are may be advantageously used.

In addition, the composition comprising *Leuconostoc* sp. strain or its culture as an active ingredient of the present invention may be used as a feed composition or feed additive composition for prevention or improvement of cancer of livestock.

When the composition is produced as a feed additive, the composition may be produced as high concentrate of 20 to 90 % or in a powder or granule form. The feed additive may additionally comprise any one, or one or more of organic acids such as citric acid, fumaric acid, adipic acid, lactic acid, malic acid, etc., or phosphates such as sodium phosphate, potassium phosphate, acidic pyrophosphate, polyphosphate (polyphosphate), etc., or natural anti-oxidants such as polyphenol, catechin, alpha-tocopherol, rosemary extract, vitamin C, green tea extract, licorice extract, keto acid, tannic acid, phytic acid, etc. When produced as feed, the composition may be formulated in a common feed form, and may comprise common feed components together.

The feed and feed additive may further comprise grain, for example, powdered or crushed wheat, oat, barley, corn and rice; plant protein feed, for example, feed having rape, bean and sunflower as a main component; animal protein feed, for example, powdered blood, meat meal, bone dust and fish meal; sugars and dairy products, for example, dried components consisting of various kinds of powdered milk and milk serum powder, and the like, and in addition thereto, may further comprise a nutritional supplement, a digestion and absorption enhancer, a growth promoting agent, and the like.

The feed additive may be administered alone or administered in combination with other feed additive in an edible carrier to animals. In addition, the feed additive may be mixed as top-dressing or directly to animal feed, or easily administered to animals as a separate oral formulation from feed. When the feed additive is administered separately from animal feed, as well-known in the art, it may be produced as an immediate release or sustained release formulation, in combination to a pharmaceutically acceptable edible carrier. This edible carrier may be solid or liquid, for example, corn starch, lactose, sucrose, bean flake, peanut oil, olive oil, sesame oil and propylene glycol. When a solid carrier is used, the feed additive may be a tablet, capsule, powder, troche, lozenge, or non-dispersed form of top-dressing. When a liquid carrier is used, the feed additive may be a formulation of gelatin soft capsule, or syrup or suspension, emulsion or solution.

Furthermore, the feed and feed additive may contain a supplement, for example, a preservative, stabilizer, wetting agent or emulsifier, solution promoter, or the like. The feed additive may be used by adding it to animal feed by salivating, spraying or mixing.
The feed or feed additive of the present invention may be applied to many animal feeds including mammals, poultry and fish.

It may be used for pigs, cows, sheep, goats, experimental rodents and pets (e.g.: dogs, cats) in addition to experimental rodents, and the like, as the mammals, and may be used for chickens, turkeys, ducks, geese, pheasants, and quails, and the like, as the poultry, and may be used for trout, and the like as the fish, but not limited thereto.

### Example Embodiment 1. Isolation and identification of strains

After crushing various types of kimchi samples, serial dilution (10³-10⁸) of the kimchi solution with PBS (phosphate buffered saline) was performed, and the dilution was plated on MRS, the primary lactic acid bacteria selection medium, and they were cultured at 30°C or 37°C for 24 hours, respectively. After the formed colonies were separated into single colonies, they were line-plated on phenylethyl alcohol sucrose agar medium, which is a *Leuconostoc* selection medium and cultured at 20°C for 24 hours. After culturing, the formed single colonies were collected. They were put and cultured in MRS broth. Genomic DNA was extracted from the culture medium cultured for 24 hours. To confirm the 16S rDNA base sequence, the obtained genomic DNA product was requested for analysis by Macrogen. The confirmed nucleotide sequence was analyzed for similarity using the Basic Local Alignment Search Tool (BLAST) of the National Center for Biotechnology Information (NCBI, www.ncbi.nlm.nih.gov), and through this result, it was finally named *Leuconostoc mesenteroides* LB-LM1 strain, and was deposited at the Korean Culture Center of Microorganisms on April 24, 2020.

### Example Embodiment 2. Culture of the strain

The isolated *Leuconostoc mesenteroides* LB-LM1 strain was inoculated with 1% in 30 ml of MRS broth and stationary-cultured at 37°C for 6 hours. After culture, the culture medium was removed by centrifugation at 3500 rpm for 10 minutes, and the cells were washed three times with a phosphate buffered saline (PBS) solution to remove the remaining medium components.

### Example Embodiment 3. Conditions of experimental animals (mouse)

As an experimental animal used in the experiment, a 5-week-old male BALB/c mouse (Orient Bio, Korea) was supplied. Mice were bred during the experiment after a oneweek stabilization period in an animal breeding room in an SPF environment where room temperature 20±2°C and humidity 55±15% were maintained.

As the feed, a normal pellet feed without antibiotics was supplied, and water was provided at any time. Observation of tumor size change was carried out by measuring the tumor volume (mm³) using the formula of 3.14 × (length × height × width) / 6.

### Example Embodiment 4. Analysis of antitumor effect in colorectal cancer CT26 cell transplantation mouse model

### 4-1. Cell culture

CT26 colorectal cancer cells were purchased from the Korea Cell Line Bank, and cells were cultured in DMEM medium supplemented with 10% FBS (fetal bovine serum) and 1% antibiotics (penicillin/streptomycin) at 5% CO₂ and 37°C conditions.

### 4-2. Preparation of CT26 cell transplantation cancer animal model

6-week-old BALB/c mice (18-21 g) were used in the experiment for the CT26 cell transplantation animal model production. 1×10⁵ cells of cultured colorectal cancer cells CT26 were harvested, resuspended in 50 µl of PBS, and injected subcutaneously into the right thigh of the mouse.

### 4-3. Analysis of antitumor effect

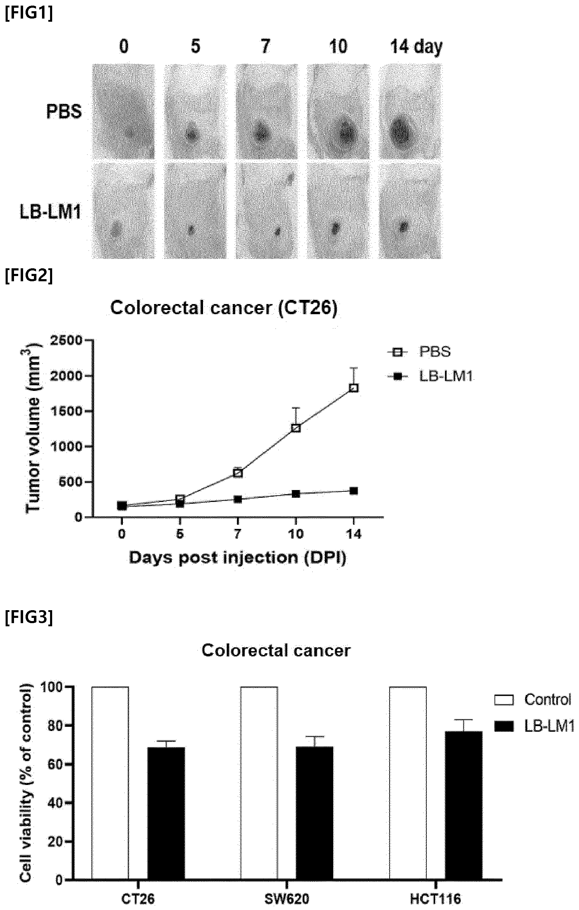

*Leuconostoc mesenteroides* LB-LM1 was intravenously injected into the tail of a CT26 cell-transplanted mouse in which the tumor was formed in a volume of about 80-100 mm³. 0.1 ml (1×10⁹ CFU) of *Leuconostoc mesenteroides* LB-LM1 prepared in PBS was intravenously injected, and PBS was administered to the control group. The results of visually confirming the change in the tumor size of the CT26 cell transplanted mice over time are shown in FIG. 1.

As shown in FIG. 1, it was observed that the group administered with *Leuconostoc mesenteroides* LB-LM1 significantly reduced the size of the tumor after 14 days compared to the control group (PBS).

In addition, the result of measuring the volume change of the tumor in the CT26 cell transplanted mice over time is shown in FIG. 2.

As shown in FIG. 2, it was confirmed that the tumor size of the CT26 cell transplanted mice was reduced by about 80% in the group administered with *Leuconostoc mesenteroides* LB-LM1 compared to the control group.

### Example Embodiment 5. In vitro anticancer activity efficacy analysis

### 5-1. Preparation of cell lines

In order to observe the anticancer activity effect of the *Leuconostoc mesenteroides* LB-LM1, the cell growth rate (cell viability assay) was measured using Cell counting Kit-8. The cells used for the anticancer activity efficacy experiment were colorectal cancer CT26, SW620, HCT116 cell lines, pancreatic cancer AsPC-1 cell line, bladder cancer T24 cell line, melanoma B16-F10 cell line, and normal skin cell line CCD-986sk.

The SW620, H1650, AsPC-1, and T24 cell lines were subcultured under the conditions of 5% CO₂, and 37°C using an RPMI medium in which 10% FBS (fetal bovine serum) and 1% antibiotics (penicillin/streptomycin) were added. The CT26, HCT116, and B16-F10 cell lines were subcultured under the conditions of 5% CO₂, and 37°C using a DMEM medium in which 10% FBS (fetal bovine serum) and 1% antibiotics (penicillin/streptomycin) were added. The CCD-986sk cell line was subcultured under the conditions of 5% CO₂, and 37°C using an IMDM medium in which 10% FBS (fetal bovine serum) and 1% antibiotics (penicillin/streptomycin) were added. The cells in the growth period were used for the experiment. The cultured cells were treated with 0.25% trypsin-EDTA in an incubator at 37°C for 3 minutes to detach the cells, and then they were washed with DPBS twice and prepared to allow each cell to be 80% confluency in a 96-well plate.

### 5-2. Cell growth rate (cell viability assay) measurement result

In the present disclosure, colorectal cancer (3 types), pancreatic cancer (1 type), bladder cancer (1 type), melanoma (1 type) cells and normal skin cells (1 type) were cultured and then were treated with *Leuconostoc mesenteroides* LB-LM1 strain to measure cell growth (cell viability).

For the cell growth rate (cell viability assay), cells were treated with *Leuconostoc mesenteroides* LB-LM1 strain at a concentration of MOI 1 and then cultured for 72 hours. After culturing, they were treated with Cell counting Kit-8 reagent, and then the absorbance (OD 450 nm) was measured. Based on the absorbance of cells not treated with *Leuconostoc mesenteroides* LB-LM1 strain, the growth rate of the cells treated with the *Leuconostoc mesenteroides* LB-LM1 strain was calculated. The results are described below.

Colorectal cancer CT26, SW620 and HCT116 cell lines were treated with *Leuconostoc mesenteroides* LB-LM1 strain to measure the cell growth rate is shown in FIG. 3.

As shown in FIG. 3, the cell growth rate of the CT26, SW620, and HCT116 colon cancer cell lines treated with the *Leuconostoc mesenteroides* LB-LM1 strain was reduced by 31.2%, 30.9%, and 22.7%, respectively. It can be confirmed that the *Leuconostoc mesenteroides* LB-LM1 strain has excellent anticancer activity against colorectal cancer.

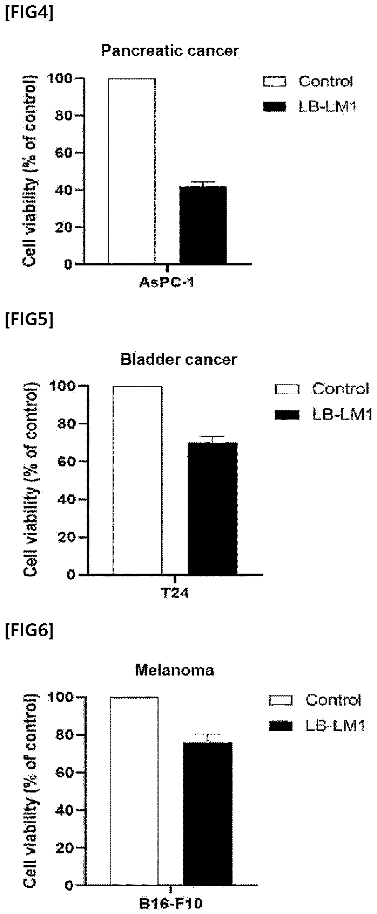

In addition, the pancreatic cancer AsPC-1 cell line was treated with *Leuconostoc mesenteroides* LB-LM1 strain to measure the cell growth rate is shown in FIG. 4.

As shown in FIG. 4, the cell growth rate of the AsPC-1 pancreatic cancer cell line treated with *Leuconostoc mesenteroides* LB-LM1 strain was reduced by 58.1%. It can be confirmed that the *Leuconostoc mesenteroides* LB-LM1 strain has excellent anticancer activity against pancreatic cancer.

In addition, the bladder cancer T24 cell line was treated with *Leuconostoc mesenteroides* LB-LM1 strain to measure the cell growth rate is shown in FIG. 5.

As shown in FIG. 5, the cell growth rate of the T24 bladder cancer cell line treated with *Leuconostoc mesenteroides* LB-LM1 strain was reduced by 29.8%. It can be confirmed that the *Leuconostoc mesenteroides* LB-LM1 strain has excellent anticancer activity against bladder cancer.

In addition, the melanoma B16-F10 cell line was treated with *Leuconostoc mesenteroides* LB-LM1 strain to measure the cell growth rate is shown in FIG. 6.

As shown in FIG. 6, the cell growth rate of the B16-F10 melanoma cell line treated with *Leuconostoc mesenteroides* LB-LM1 strain was reduced by 23.9%. It can be confirmed that the *Leuconostoc mesenteroides* LB-LM1 strain has excellent anticancer activity against melanoma.

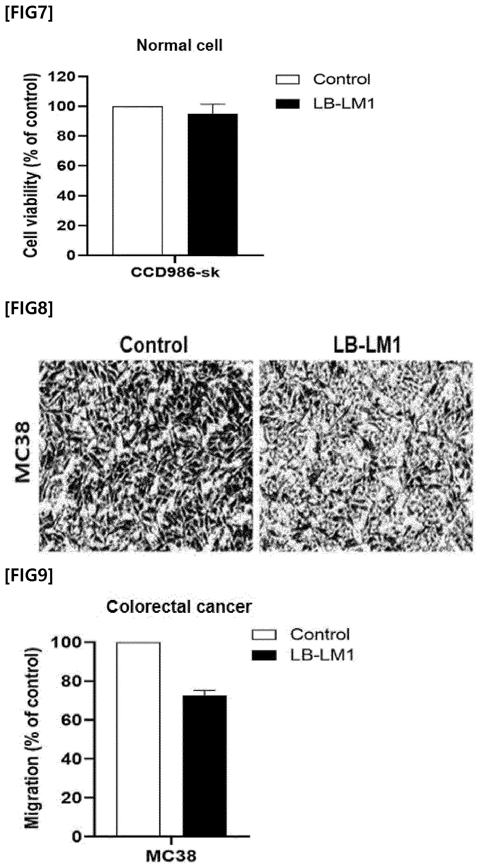

In contrast to the above experiment, normal skin CCD-986sk cell lines were treated with the *Leuconostoc mesenteroides* LB-LM1 strain, and the results of measuring the cell growth rate are shown in FIG. 7.

As shown in FIG. 7, it can be confirmed that the *Leuconostoc mesenteroides* LB-LM1 strain does not show toxicity to the CCD-986sk normal skin cell line.

### Example Embodiment 6. Confirmation of In vitro metastasis (migration assay) inhibitory ability

### 6-1. Preparation of cell lines

In order to confirm the metastasis inhibiting ability of the isolated *Leuconostoc mesenteroides* LB-LM1 strain, MC38, a colorectal cancer cell line, was used. After preparing MC38 cells to become 2×10⁶ cells in a 100 mm culture dish, the *Leuconostoc mesenteroides* LB-LM1 strain was treated with MOI 1, and cultured for 24 hours at 5% CO₂, 37°C conditions. After culture, cells were detached by washing twice with PBS and treating with 0.25% trypsin-EDTA. The outside of the trans-well chamber membrane was precoated with 10 µg/ml fibronectin, and the detached cells were put into the chamber and cultured for 24 hours at 5% CO₂ and 37°C conditions. After culture, they were washed twice with PBS and stained with Diff Quik solution. After drying sufficiently, images were taken under a microscope to measure the number of cells to calculate the metastasis results.

FIG. 8 shows a photograph taken after the colorectal cancer MC38 cells treated with *Leuconostoc mesenteroides* LB-LM1 strain were metastasized.

As shown in FIG. 8, it was confirmed that the MC38 colorectal cancer cell line treated with the *Leuconostoc mesenteroides* LB-LM1 strain was reduced in the number of cells that migrated through the chamber compared to the control.

In addition, the metastasis rate calculated by treating the colon cancer MC38 cell line with the *Leuconostoc mesenteroides* LB-LM1 strain is shown in FIG. 9.

As shown in FIG. 9, it was confirmed that the metastasis rate of the MC38 colorectal cancer cell line treated with the *Leuconostoc mesenteroides* LB-LM1 strain was reduced by 27.5%. It was confirmed that the *Leuconostoc mesenteroides* LB-LM1 strain had excellent efficacy in inhibiting metastasis of colorectal cancer.

### [Accession number]

Name of deposit institution: Korean Culture Center of Microorganisms (Overseas)
Accession number: KCCM12701P
Deposit date: 20200424

## Claims

1. A pharmaceutical composition for preventing or treating cancer, the composition comprising *Leuconostoc* strain or a culture thereof as an active ingredient.

2. The pharmaceutical composition for preventing or treating cancer of claim 1, wherein the *Leuconostoc* strain is *Leuconostoc mesenteroides, Leuconostoc carnosum, Leuconostoc citreum, Leuconostoc holzapfelli* or *Leuconostoc* lactis.

3. The pharmaceutical composition for preventing or treating cancer of claim 2, wherein the *Leuconostoc mesenteroides* strain is *Leuconostoc mesenteroides* LB-LM1 (accession number KCCM12701P).

4. The pharmaceutical composition for preventing or treating cancer of claim 1, wherein the cancer is colon cancer, pancreatic cancer, bladder cancer, skin cancer or melanoma.

5. The pharmaceutical composition for preventing or treating cancer of claim 1, wherein the composition can be administered by the method of oral administration or parenteral administration.

6. The pharmaceutical composition for preventing or treating cancer of claim 5, wherein the method of parenteral administration is intravenous injection, subcutaneous injection, intramuscular injection, intraperitoneal injection, endothelial administration, topical administration, intranasal administration, intrapulmonary administration, or rectal administration.

7. A food composition for preventing or alleviating cancer, the composition comprising a *Leuconostoc mesenteroides* (LB-LM1, accession number KCCM12701P) strain or a culture thereof as an active ingredient.

8. The food composition for preventing or alleviating cancer of claim 7, wherein the food is a health functional food.

9. The food composition for preventing or alleviating cancer of claim 7, wherein the food is any one food selected from the group consisting of bread, rice cake, candy, chocolate, gum, ice cream, milk, cheese, processed meat products, processed fish meat products, kimchi, soy sauce, soybean paste, gochujang, black soybean paste, cheonggukjang, vinegar, ketchup, curry, dressing, beverage, and fermented milk.

10. A food additive composition for preventing or alleviating cancer, the composition comprising a *Leuconostoc mesenteroides* (LB-LM1, accession number KCCM12701P) strain or a culture thereof as an active ingredient.

11. A feed composition for preventing or alleviating cancer of livestock, the composition comprising a *Leuconostoc mesenteroides* (LB-LM1, accession number KCCM12701P) strain or a culture thereof as an active ingredient.

12. The feed composition for preventing or alleviating cancer of livestock of claim 11, wherein the livestock is any one selected from the group consisting of pig, cow, horse, sheep, rabbit, goat, rat, hamster, guinea pig, dog, cat, chicken, turkey, duck, goose, pheasant, quail, carp, crucian carp and trout.

13. A feed additive composition for preventing or alleviating cancer of livestock, the composition comprising a *Leuconostoc mesenteroides* (LB-LM1, accession number KCCM12701P) strain or a culture thereof as an active ingredient.
